# EUROPEAN PATENT SPECIFICATION

(11) **EP 2 231 013 B1**
(45) Date of publication and mention of the grant of the patent: **30.11.2011**
(21) Application number: 09702534.0
(22) Date of filing: 14.01.2009
(51) Int. Cl.: A61B 5/11, G06F 19/00

(54) **INTEGRATED UNIT FOR MONITORING MOTION IN SPACE**
INTEGRIERTE EINHEIT ZUR ÜBERWACHUNG VON BEWEGUNGEN IM RAUM
UNITÉ INTÉGRÉE POUR SURVEILLER LE MOUVEMENT DANS L'ESPACE

(30) Priority: 16.01.2008 US 21373
(43) Date of publication of application: 29.09.2010
(73) Proprietor: Syddansk Universitet, 5230 Odense M (DK)
(72) Inventor: VOSS, Frands, DK-6400 Sonderborg (DK); ANDERSEN, Niels, DK-6440 Augustenborg (DK)
(74) Representative: Orsnes, Henrik Egede
(86) International application number: PCT/EP2009/050398
(87) International publication number: WO 2009/090200

(56) References cited:
- WO-A-2007/082389
- US-A1- 2007 032 748
- US-A1- 2007 129 769

## Description

### FIELD OF THE INVENTION

The present invention concerns a method for measuring the 3D-movement of one or more body parts of a human and a device for accomplishing the method. Specifically, the method and device of the present invention make use of a novel signal filtering procedure, which significantly improves the precision of present device in relation to prior art devices.

### BACKGROUND OF THE INVENTION

Conventional technologies for measuring motions of human bodies or the like are classified into i) methods of installing a sensor in a place away from a measuring target to measure it (method by remote measurement), and ii) methods of attaching a sensor to a measuring target itself to measure its motion without relying on a signal or the like from the outside (method by autonomous measurement).

Examples on methods by the remote measurement are methods of using an optical image sensor (e. g., JP 2000-182058 A, JP 2002-8043 A, or JP 10-74249 A). As the methods by the remote measurement can execute measurement only in a space in which the sensor installed outside is functional, its measuring range is limited. Depending on a sensor type, there is a space in which the sensor cannot be installed. For example, a method of tracking by the optical image sensor with an LED or the like as a mark cannot be used outdoors when it is bright. A method of tracking a marker attached to a measuring target by applying an infrared light (e.g., VICON by Vicon Motion Systems Inc.) cannot be used outside, either.

The method by the autonomous measurement has an advantage of no restrictions on a measuring range. Measuring methods are so-called mechanical types to measure elongation/contraction of a wire, a change in an angle of a bar, relative distances among sensors attached to the limbs, or the like (e.g., by Gypsy Spice Inc.), and they are similar in that joint angles are measured.

All the methods need to attach the sensor to each limb, restricting a joint motion itself in many cases. As the sensor itself is large, its appearance gives an uncomfortable feeling, and thus the sensor is not suitable for outdoor use. Accuracy of angle measurement is low, and thus unsatisfactory for highly accurate operation measurement.

Micro Electro-Mechanical System (MEMS) technology has resulted in the development of a micro, low-cost Inertial Measurement Unit (IMU) that comprises MEMS angular rate and acceleration sensors on silicon chips. However, despite their enormous cost, size, weight, thermal stability, and wide dynamic range advantages over conventional inertial sensors, the MEMS IMU generally yield less precise and accurate measurements relative to their conventional IMU counterparts.

It is also known to use a high-precision positioning and data integrated system comprising a MEMS IMU, two GPS systems providing raw data, and a centralized processing module that comprises Kalman filter integration to correct various measurement errors such as tilt angle shifts, velocity error, and accelerometer errors received by the device's navigation module. However, GPS systems may be blocked by obstructions, thereby reducing the effectiveness of the system.

The concept of a "virtual gyro" has also been explored, wherein gyroscopes are replaced with an array of relatively inexpensive accelerometers. The system comprises distributed accelerometers combined with a multiple-antenna, GPS-based, attitude- determination system. In this system, however, accelerometer noise is known to be a major source of errors in both angular and linear parameters.

One critical cause of error in position measurement is error in the orientation determined by the gyros. This is so because the maturity of MEMS accelerometers, now in the 100-mg class, has put extreme pressure on the pitch and roll accuracy to reach the same level of performance, which is far more difficult for the gyros than for the accelerometers.

Thus, it is not impossible now to attach the sensor to the body thereby measuring a human posture. However, to calculate a moving distance or a direction from an output of the inertia sensor, integration must be carried out twice in the case of the acceleration sensor, and once in the case of the angular velocity sensor (gyro-sensor). Accordingly, small errors accumulate with time caused by a fluctuation in a still output which accompanies electric noise in the sensor output, a shift in gravity axis, or a change in a surrounding environment such as a temperature. A drift phenomenon occurs in a measuring position even in a still state. As a method of correcting this drift, use of an external signal of an ultrasonic wave, magnetism, a light, or the like is general. For example, in motion tracking (refer to US 6,176, 837 and 6,474,159), an instrument for determining a direction or a position of a body, or a head mount display instrument for reproducing a human posture in a virtual space of a computer is realized by a system for correcting an error of an inertia measuring instrument by an ultrasonic wave. However, those methods impose spatial restrictions after all as in the case of the aforementioned method by the remote measurement. An attempt has been made to measure a joint angle of a human body only by an inertia sensor (refer to JP 11-325881 A). However, there are restrictions in that the sensor must be attached as close as possible to both ends of the joint, and usable places are limited to a hand, a leg, and the like.

Measurement of a motion of a human body is necessary in many fields. In management engineering, there is an example of measuring and analyzing a working motion of a worker in detail to improve working efficiency. In computer graphics, to represent a real human motion, the human motion must be accurately measured. In a medical field, measurement of a motion must be accurately carried out to quantitatively understand how a motion of a patient is improved in a process of rehabilitation or the like. Thus, the human body motion measuring system is expected to be used for various purposes, and some measuring systems have been developed. However, in measurement, most of the systems inevitably impose restrictions on human behaviors which become measuring targets.

Body motion tracking is often limited to laboratories equipped with camera-based tracking systems (see, for example, systems developed by Human Performance Labs, and area-limited performer-trackers, such as those of Ascension MotionStar). Because of the complex optical environment required, measurements outside of the laboratory are notoriously difficult. Portable solid-state angle sensors such as those employing accelerometers - although accurate for static measurements - are not suitable for body motion tracking. They are highly sensitive to the accelerations associated with normal human movement. There are inherent problems with trying to measure relative position and absolute velocity with these types of sensors without correcting for inertial effects (in the case of accelerometers) and integration offset (in the case of gyroscopes).

WO 2007/082389 A and US 2007/032748 A1 disclose an accelerometer component operative to perform acceleration measurements along 3 orthogonal axis, a gyroscopic component operative to measure rotational velocity along the 3 orthogonal axes, and a magnetometer component operative to perform magnetic measurements along the 3 orthogonal axes. Moreover, WO 2007/082389 A and US 2007/032748 A1 disclose a data memory unit connected to the components, said data memory unit used for receiving and storing data from the components, a power supply, a timer for synchronizing the operation of the components, and a timer for synchronizing the operation of the components and means for attaching the unit to the skin of a human.

Meanwhile, the units or devices disclosed in the prior art, including WO 2007/082389 A and US 2007/032748 A1, need further improvements in order to be comfortably worn by the user, and the sensor components need to be further stabilized and protected, e.g. from humidity.

Accordingly, there is a need in the art for a comprehensive tool to augment capacity and disability testing by sensing body positions and movements over extended periods of time. A portable device that could be attached to a part of the body and accurately measure its orientation could have numerous applications.

With the foregoing in mind, the present invention provides a system which can correct the aforementioned drift phenomenon of the measuring position and execute efficient measurement with minimum motion restrictions (e.g., not only indoor but also outdoor free spaces).

### SUMMARY OF THE INVENTION

The present invention provides an integrated unit for determining the position of a moving part, such as a body part, comprising:
- an accelerometer component operative to perform acceleration measurements along 3 orthogonal axes;
- a gyroscopic component operative to measure rotational velocity along said 3 orthogonal axes; and
- a magnetometer component operative to perform magnetic measurements along said 3 orthogonal axes;
- a data memory unit for receiving and storing data from said components;
- timer for synchronizing the measurements of the components; and
- optionally a data processing unit in the form of a computer, said computer implementing a kalman filtering routine or other validating filtering routine, for processing said data to accurately determine positions of said components;
wherein said components, data memory unit, and optionally said data processing unit are embedded in a polymer matrix, such as silicone, which is provided with means for attaching the unit to the moving part.

The means for attaching the unit to the skin of a human may be an adhesive layer, such as an adhesive silicone. Preferably the data memory unit is capable of relaying said data to the data processing unit in real time; for example the data processing unit may be provided in the vicinity of the integrated unit (and thus the memory unit) and thereby wirelessly receive the data to be processed.

When used to monitor the movement of a body part sensors may also be placed under each foot to measure weight and pressure. The foot sensors are able to sense pressure in both the heel and ball of the foot. The foot sensors precisely measure applied pressure. Depending on the way the patient's weight is distributed on his or her feet, the pressure reading may or may not directly translate to an accurate weight reading, however, the pressure readings will be consistent. The foot sensors 15 are flat. Each foot sensor 15 will consist of an ankle "bracelet" (attached to the ankle with a Velcro® strap) containing the electronics and an attached flexible "tongue". The tongue contains the pressure sensors to be placed under the sole of the foot, normally inside a shoe.

The integrated sensor unit of the present invention comprises magnetometers, accelerometers, timers and gyroscopes. The integrated sensor unit provides for accurate measurement of body position. The gyroscope angular velocity measurements - unaffected by acceleration - are used to continuously rotate a matrix that represents the orientation of the sensor. The magnetometer and accelerometer measurements are used to construct a second matrix that also estimates the sensor's orientation - unsusceptible to the drift introduced by integrating the gyroscope signals. The first matrix is "pulled" slightly towards the second matrix each step of the computation, thereby eliminating drift.

The integrated sensor unit performs acceleration measurement along 3 axes, inertial (gyroscopic) measurement along 3 axes, and magnetic measurement along 3 axes. Several different embodiments of the 3D sensor unit are contemplated. For example, since some commercially available accelerometers and magnetometer chips have 2 axes per chip, one economical embodiment of the 3D sensor is made up of 2 accelerometers, 3 gyroscopes, and 2 magnetometers (i.e. only 3 of 4 available accelerometer and magnetometer axes would be used). Alternatively, the 3D sensor could be built using 3 accelerometers, 3 gyroscopes and 3 magnetometers, each having one axis. In each of these embodiments the 3D sensor is capable of performing acceleration, gyroscopic and magnetic measurements along 3 axes.

The present invention can be used for a position determining instrument equipped with at least an acceleration sensor, a gyro-sensor and a magnetic field sensor. According to the present invention, based on an output from the acceleration sensor and gyro-sensor, a vector direction is determined.

A basic posture of the measuring target is defined beforehand on the coordinate system, and a sensor attaching position is specified further on the definition. If there is no error in a value from the sensor, the motion can be accurately measured. However, an output fluctuation caused by a temperature change or the like is accompanied by a drift phenomenon. Accordingly, the method of the present invention is applied to correct the deviation. Assuming that a direction of gravity is always constant on Earth, a direction of gravity determined by the acceleration sensor of the still state means that a measured value always indicates accurate direction of gravity.

According to the present invention, a system may be employed to capture an output from the sensors into the computer by wire. Preferably, however, a transmitter is disposed in the sensor unit, and a receiver is disposed on the computer side, whereby the sensor output is wirelessly taken into the computer. This is preferable because it imposes no restrictions on the motion of the measuring target.

According to the present invention the accuracy is increased when a magnetic direction sensor is provided, an initial magnetic direction is determined by the magnetic direction sensor, the initial magnetic direction is changed associatively with the motion of the moving body part, a magnetic direction is then measured by the magnetic direction sensor after a passage of optional time, an error is measured based on a difference between the after-motion magnetic direction and the magnetic direction after the passage of the optional time, and the error is corrected associatively with error measurement in a direction of gravity by the acceleration sensor. Accordingly, motion of the human body can be measured.

In contrast to prior art solutions the present integrated unit of the present invention can be placed directly on the body part, which motion is to be measured. Accordingly, the number of attached sensors can be reduced as compared with the prior art methods designed for joint angle measurement, and a free joint motion is not restricted. The sensor itself can be miniaturized, and thus its attachment is easy.

Thus, the present invention is applicable to e.g. acquisition of three-dimensional data for medical treatment and sports. A state of caring/rehabilitation/work (posture) is measured and represented by a numerical value. This is a system for measuring a motion of a patient to check a treatment effect or quantitatively understand a progressing situation, thereby supporting technique improvement or the like. This is also a system for analyzing a three-dimensional motion of an athlete or a dancer, thereby supporting improvement of techniques etc.

Specifically, the present invention provides a self-contained, integrated micro-sized unit in which component sensors can provide changes in angular rate and velocity of a body part to high accuracy. The accuracy is achieved through the use of specifically oriented accelerometer, gyro and magnetic field sensors, the orientation serving to substantially cancel noise and other first-order effects, and the use of de-noising algorithms to reduce the noise of MEMS gyro and accelerometer devices, and an error-correcting algorithm such as a Kalman filter embedded in a digital signal processor (DSP) device. In an additional DSP, a navigation-grade Kalman filter algorithm couples to the IMU to provide navigation-grade performance results.

The integrated unit of the present invention also comprises a magnetometer for determining the down position of the navigational carrier to avoid orientation problems. The fully integrated unit of the present invention is lightweight and compact in size for mounting on individuals, such as soldiers, to detect movement, as well as to determine instantaneous motion change and new position on low-cost stabilization platforms.

It is important that the unit is attached to well selected places, for example in body places, in which bone structures are in close proximity to the skin surface, i.e. with no or only small muscle or fat layers are covered.

The use of acceleration sensors, which measure the static acceleration of the force of gravity, makes the determination possible of the initial directions with static initial positions. Otherwise initial movements must be measured (and possibly over a multiplicity of measured values to be averaged). The vertical direction can be determined thereby for example by a short jump.

The present invention allows for the precise and non-invasive measurement of knee motion and its analysis in 3D. Specifically, the present invention measures precisely and non-invasively the relative 3D position and orientation of the tibia in respect with the 3D position and orientation of the femur during time and the relative 3D movement of the tibia in respect of the femur; this is achieved by attaching an integrated unit of the present invention on the skin of the upper and lower leg; the 3D data gathered in the integrated units through the motion of the leg are subsequently transferred to a computer, whereby the relative spatial motion of the upper and lower leg can be assessed.

In this context it is worth to note that human joints are usually more complex than a single axis. The knee joint is among the most complicated synovial joints in the musculoskeletal system. The kinematic studies of knee allow the computation of force distribution during physical activities (such as walking), evaluating surgical operations such as ligament reconstruction, evaluating the effects of inaccurate positioning of condylar prostheses, evaluating the effect on the knee of the use of foot prosthesis, evaluating diagnostic methods for injuries and studying the injury mechanism in a knee joint.

By performing a combination of rolling and sliding, the knee joint accommodates the small contact area between the femur and the tibia. The anatomical structure of the femoral condyles leads to a complex combination of translations and rotations, which includes components of abduction or adduction, internal or external rotations and flexion or extension. To measure the rotations, localising sensors (magnetic, optic, ultrasonic) can be used in order to follow the position and orientation of the femur and the tibia in space. Experiments have been made in order to measure the relative motion between the femur and the tibia using such sensors placed on the skin.

However, Macleod and Morris (1987) were the first to study the inevitable relative movement between skin and bone during a movement analysis. This has also been done by Sati et al. (1996) who has reported three general methods which address the problem of relative skin movement: 1) use of intra-cortical pins to fix rigidly but invasively the sensors to the bones, 2) use of statistical calculations to correct the positions of several sensors and 3) use of attachment systems in order to reduce sensors movement in respect with the underlying bone. Only the third method allows having relatively precise measurements of the bone position and orientation, non-invasively. Because these two factors are essential during routine examinations of the knee, the use of an external attachment system seems to be the best compromise.

Sati et al. (1996) proposed an attachment system for the sensors. This mechanical fixation system 5 attaches the sensors onto the underlying bone non-invasively. Three attachment sites onto the condyles are related with a mechanical bridge, which insure the application of inward clamping pressure. A vertical bar insures the system to accurately reflect the orientation of the femoral long axis. The tibial attachment consists of a long bow-shaped plate strapped at both ends to the proximal and distal ends of the tibia. it has been shown that the system can measure knee kinematics with acceptable precision (Sati et al. 1996). This attachment system however revealed some problems in its use:
The mechanical bridge which relates the attachment sites on the femoral harness is designed to be flexible in order to provide comfort to the subject when performing extension of the knee since biceps femoris tendon and ilio-tibial band approach one another during full extension, and the lateral attachment sits on the biceps femoris muscle which has the effect of pushing the lateral attachment away from the knee (Sati, 1996).

However, this causes a displacement of the three femoral attachments, particularly on the lateral side, that produces an antero-posterior force which can lead to harness detachment. Also, the localising sensors motion is then influenced by their location on the attachment system. Moreover, the mechanical bridge flexibility causes orientation changes in part of the harness during subject full extension, which can result in errors in measurements of the position and orientation of the sensors fixed on the harness. Further, the addition of force exerted on knee structures when performing full extension is similar for all subjects. Although it can be acceptable for many subjects, the force can be unbearable for some. Finally, the adjustment and installation is somewhat long and not precise. A second version of the harness was produced, with a bridge that is rigid in expansion but flexible in torsion, relating one lateral and two medial supports. No lateral expansion is possible during knee extension because of the bridge's rigidity in expansion,which produces an unbearable pressure on both sides of the knee for most of the subjects and causes errors in measurements.

Due to these disadvantages, there is also a need to provide a new design in order to improve the precision, the sensibility and the reproducibility of the knee analysis system without affecting the subject's comfort.

Use of the present invention permits precise measurements and analysis of the knee movement, i.e. the description during time of the tibial and femoral three-dimensional positions and orientations, one with respect to the other.

It is a further feature of the present invention to provide a harness which can obtain a non-invasive attachment for the localising sensors on the femur, which is composed of orthoplasts, not related by a flexible mechanical bridge, and which is comfortable for the subject, especially during full extension.

In accordance with the above features, in a broad aspect, the present invention allows for a knee movement analysis method comprising placing, in a non-invasive manner, of 3D magnetic field sensors on the femur, and onto the tibia, and applying a program for analysing the location measurements, therewith providing results on kinematic or posture of the knee, characterised in that the sensors are attached to the skin by applying an adhesive.

The present invention can be used as a three-dimensional knee movement analysis system, which uses an adhesive for localising 3D magnetic field sensors on the femur and on the tibia. The lateral movement of the skin onto which the sensors are attached may be compensated for by a calibration routine, which filters the skin movements/vibrations via focus on harmonic skin movements and wavelet theories.

The customized filtering of skin movements ensures that analysed data can be filtered and hereby producing a more accurate reading. The skin movements of a person are unique and therefore the filtering has to be dynamic since fat and muscle add to the wobbling effect of the skin.

### DETAILED DESCRIPTION OF THE INVENTION

A description of the preferred embodiments of the present invention will now be presented. The device of the present invention includes a self-contained, integrated unit in which gyros and accelerators along with magnetic field sensors provide high-accuracy position and velocity. The integrated unit of the present invention is attached to the skin via an adhesive layer comprising a skin tolerant adhesive, such as Sensura from Coloplast. Instead of using an adhesive layer a vacuum can be created inside a suction cup placed on the skin of a person, where the unit is attached to the suction cup upper or lower part or integrated within the suction cup. By placing the unit inside the suction cup, the unit will be pressed against the skin of the person. The vacuum can be built by using a pump or compressor connected to or in connection with the suction cup or by an integrated vacuum pump in the unit, which may use vibrations from the movement of the person, where it is mounted to ensure a proper level of vacuum. Such a unit should be set in place on the skin of a person and shook, before initial vacuum is created.

A complementary Kalman filter estimates orientation, gyroscope offset, and magnetic disturbance by combining 3D gyroscope, accelerometer, and magnetometer signals using relevant signals. The direction of the global magnetic vector is estimated by the gyroscopes and magnetometers. It is assumed that the magnetometer can compensate for local magnetic disturbances. As long as the disturbance is within a specified boundary, the new magnetic field vector can be used as a reference.

The Kalman filter and an output of the filter are connected to the measurement system to provide a correction signal for correction of gyro bias errors, the accelerometer errors, and the temperature derived from an internal temperature sensor to measure and correct for temperature extremes.

The proper internal data synchronization is very important for accurate signal processing. A high- precision internal time reference therefore is very important to be available inside the integrated unit. If more integrated units, placed on different body parts, are to interact with each other, a central synchronization unit must be able to communicate and synchronize all of the integrated units. All gyro conversions are simultaneously sampled, and the accelerometer is sampled at high rates. The accurate time reference is set by the internal clock of the synchronization unit. The synchronization may be set by e.g. triggering the units by an impulse, such as a light impulse (e.g. infrared), a radiofrequency wave, a jump, or similar. Such an impulse will then start a timer in the integrated units, which are then synchronized.

The Kalman filter provides an on-line calibration by providing a real- time characterization of the gyro bias state. The accelerometers provide a reference using gravity and the triaxial magnetometer senses true North while compensating for magnetic metals close to the sensor location. A Kalman filter is used to blend the magnetometer/accelerometer measurement with the estimates obtained by straight integration of the gyros.

The magnetic sensor can have significant distortions in the heading output provided by the compass due to its close proximity to the other sensors and the electronics, and thus calibration requires a substantial effort. Besides the constant magnetic declination, the compass is affected by local distortions of Earth's magnetic field. With a non-ferrous mechanical turntable used in initial calibration of the module, it is possible to measure these errors.

Moreover, a so-called event-logger (part of the data processing unit) is provided for storing the information gathered by the data processing unit. An event logger records information, such as start time, duration and sequence of events with the elapsed times when requested through a system manager. The event logger may be in wireless communication with one or more integrated units placed on the body, so as to provide dynamic information from several moving body parts in space.

A wearable trigger can be placed on the wrist of a person alike a watch, this allows the user to mark the data intervals for in depth analysis. By pressing one button the interval can be marked and unmarked for analysis. This can be used in connection with pain felt, so that felt pain can be marked for in depth analysis.

An external trigger can be placed and register when the person is closest to it by sending out radio waves and measuring when the signal is strongest. When the signal is strongest the interval can be marked.

The unit(s) enable the plotting of a movement id based upon the data values extracted from the movement of the person. The movement id illustrates the standard values the person achieves and can give a quick overview of the spread of the values. The main reason for plotting the values is to provide a customized movement id, which can be used as reference for analysis of the person in question. Focus can be placed upon stress, fatigue and/or muscle overuse. The efficient use of energy can also be motioned - from the persons movement energy can be calculated, this energy can be compared to the actual results. Hereby the efficiency of different persons can be compared and more effective training methods can be developed.

Concerning the applicable adhesives to attach the integrated unit to the skin, the preferred adhesives are silicone adhesives or modified silicone adhesives such as the polydimethylsiloxane. Meanwhile a broader range of adhesives may be used; broadly these include acrylics, silicones, polyisoalkalines, rubbers, vinyl acetates, polyisobutylene rubber, polybutyldiene, styrene-butadiene (or isoprene)-styrene block copolymer rubber, acrylic rubber, and natural rubber; vinyl-based high molecular weight materials such as polyvinyl alkyl ether, polyvinyl acetate, a partially saponified product of polyvinyl acetate, polyvinyl alcohol and polyvinyl pyrrolidone; cellulose derivatives such as methyl cellulose, carboxylmethyl cellulose and hydroxypropyl cellulose; polysaccharides such as pullulan, dextrin and agar; polyurethane elastomers; and polyester elastomers. Of course, the adhesives must be biocompatible and nonirritating. They must also allow for a patch to adhere firmly to the skin or mucosa of a patient in need of treatment by a patch, but not be so adhesive so as to injure the patient as the patch is removed. It is also important that the adhesive be selected such that it is compatible with the other components of the present invention.

Examples of the polyacrylate adhesives for use in the present invention are those sold under the trademark DuroTak®87-2194, 87-2620, 87-2052, 87-2852, 87-2054, 87-2979 and 87-6173 by National Starch and Chemical Corporation. Other suitable adhesives are sold under the trademark GELVA-Multipolymer Solution, GELVA 2873 and 2883 by Surface Specialties, Inc.; and silicone adhesives sold under the trademark EIO-PSA 7-4300 and 7-4500 by Dow Corning Corporation. Other preferred adhesives include polyisobutylene and styrene-butadiene rubber adhesives.

Examples of the copolymerizable monomer include carboxyl group- containing monomers such as (meth)acrylic acid, itaconic acid, crotonic acid, maleic acid, maleic anhydride and fumaric acid; sulfoxyl group- containing monomers such as styrenesulfonic acid, arylsulfonic acid, sulfopropyl acrylate, (meth) acryloyloxynaphthalenesulfonic acid, acrylamidomethyipropanesulfonic acid and acryloyloxybenzenesulfonic acid; hydroxyl group-containing monomers such as hydroxyethyl (meth)acrylate and hydroxypropyl (meth)acrylate; amide group- containing acrylic monomers such as (meth)acrylamide, dimethyl (meth) acrylamide, N- butylacrylamide, tetramethylbutylacrylamide and N-methylol (meth) acrylamide; alkylaminoalkyl group-containing acrylic monomers such as aminoethyl (meth) acrylate, dimethylaminoethyl (meth) acrylate, diethylaminoethyl (meth) acrylate and tertbutyl (meth) acrylate; alkyl esters of acrylic acid containing an ether bond in the molecule thereof such as methoxyethyl (meth)acrylate, ethoxyethyl (meth) acrylate, butoxyethyl (meth) acrylate, tetrahydrofurfuryl (meth) acrylate, methoxyethylene glycol (meth) acrylate, methoxydiethylene glycol (meth) acrylate, methoxypolyethylene glycol (meth)acrylate and methoxypolypropylene glycol (meth)acrylate; vinyl monomers such as N- (meth)acryloylamino acid; functional monomers such as urethane, urea or isocyanate ester of acrylic acid; and vinyl monomers such as (meth) acrylonitrile, vinyl acetate, vinyl propionate, vinyl pyrrolidone, vinyl pyridine, vinyl pyrazine, vinyl piperadine, vinyl piperidone, vinyl pyrimidine, vinyl pyrrole, vinyl imidazole, vinyl caprolactam, vinyl oxazole, vinyl thiazole, vinyl morpholine, styrene, a-methylstyrene and bis (N,N' - dimethylaminoethyl) maleate.

Concerning the polymer matrix in which the components are embedded this material preferably is a cross-linked silicone polymer. For example a cured silicone-based gel wherein the polymer matrix is a crosslinked silicone polymer is preferred, but it is envisioned that other polymer matrices could be used for the present invention, such as those based on polyurethanes, polyureas, anhydride-containing polymers and the like.

Preferably the magnetic sensor is a magnetic 3D chip from Honeywell (product no: hmc1043) having the following dimension 4x4x1.5 mm; the gyro is a 1 D gyro chip from Epson (product no: xv-3500cb); and the accelerometer is a 3D accelerometer from Analog Devices (product no: adxl330kcpz). All of these sensors are fixed on a small print-board, which also comprises the synchronization component (synchronization impulse receiver, trigger and timer).

### EXAMPLE 1

Initially the composants (Amx, Amy, Amz) of the gravitation field are measured in the local system. Since the three accelerometers of the integrated unit are not subjected to any acceleration in the local system the measured acceleration corresponds to the acceleration of gravity. This direction (vector) is denoted -Zg as the direction in the global coordinate system.

Based on magnet field measurements the coordinates of the magnetic field (Bmx,Bmy,Bmz) are defined in the local coordinate system. With these to set of measurements, namely gravitation and magnetic field (G and B), the basis for a global reference system is defined. The X-axis in the global system may be chosen to be 30 degrees twisted relative to the horizontal composant of the B-field. The direction of the horizontal composant is determined by e.g. crossing Bm with Am and then crossing this result with Am again. The angle of the global y-axis is consequently 60 degrees and the x-y plane is horizontal.

Based on the starting point each transducer is sampled and the values produced in the local system are transformed into the global system, wherein a new set of data as regards acceleration, velocity, position and orientation (direction) is determined.

The procedural steps carried out by the software program are:

| | |
|---|---|
| | Verify that the intetrated unit is static |
| | !Am! =1g =9.8m/sec² |
| | ▼ |
| | Measure the composants of Am in the local coordinate system |
| | ▼ Determine the direction for Zg in the global coordinate system |
| | |
| | ▼ |
| | Determine Bm in the local system |
| | ▼ |
| | Determine horizontal direction (perpendicular on Zg) in the local system. |
| | ▼ |
| | Define directions for Xg and Yg (axes) |
| | ▼ |
| | Define Euler angles for the global system relative to the local system |
| | ▼ |
| | Define Euler angles for the local system relative to the global system |
| | 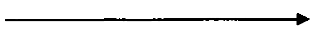 ▼ |
| 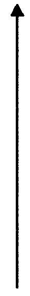 | Start calculations on motion (sampled values) |
| | ▼ |
| | The sampled values are transformed into the global coordinate system |
| | ▼ |
| | Determine new set of acceleration, velocity, position, and orientation values |
| | ▼ |
| | 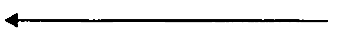 |

The result of the distance moved may be shown in a global (static) reference system.

It is important to know which data may be given importance (validation). It is well known that the earth magnetic field changes in the presence of large volumes of iron or other magnetic materials.

A procedure, which calculates the total measured magnetic field (Btot) is therefore adopted. If the measured value differs from the earth magnetic field (49.6 micro Tesla) by more than a certain percentage this value is not used for determining the orientation of the integrated unit. In the following an example is given. A sensor unit (the integrated unit of the present invention) is turned with a constant angular velocity of 3.14 rad/sec in the global system around the Zg axis.

With reference to Tables 1 and 2 the principles of the measurements are herewith outlined:
Bm: field in micro Tesla
   Sample = time lapse
   Sample 8 to 20 have too high Btot values (due to external influence)
   These values are not used to calculate the orientation.
Bx, By and Bz are the measured values in the local system (micro Tesla)
   Btot is the calculated !Bm!= square root of Bmx²+ Bmy²+ Bmz²
   In this embodiment the treshold is set to +/- 10%
   Pxg, Pyg and Pzg are calculated position in the global system (meter)
   Ksi, Fi and Theta are Euler angles indicating the orientation (radians)
   Axg, Ayg and Azg constitute the acceleration values converted into the global system

In the sample period 5-15 the sensor unit is subjected to an acceleration beyond the acceleration of gravity in the direction of the y-axis.

The start velocity is set to zero. Accordingly Px and Pz remain unchanged (except for noise contributions). This can also be applied when the acceleration is 1g in the Zg direction (where the unit is static or has a constant velocity). Starting angles Ksi=Fi=Theta=pi/4=45 grader, twisting only occurs around the Zg axis in the global system. From timestep 5-15 the acceleration is 0.4g in y-axis direction + gravitation in z-axis.

**Table 1**

| Output from measurement | | | | | | | | | | | |
|---|---|---|---|---|---|---|---|---|---|---|---|
| Time | Amx | Amy | Amz | Omgx | Omgy | Omgz | Bmx | Bmy | Bmz | Vv | Temp |
| 1.0000 | 0.4989 | 0.5004 | 0.7066 | 1.5689 | 1.5628 | 2.2219 | 32.5906 | 9.0073 | 35.8529 | 3.7047 | 19.3080 |
| 2.0000 | 0.5017 | 0.4991 | 0.7085 | 1.5700 | 1.5543 | 2.2003 | 29.7309 | 7.4745 | 40.3099 | 3.7070 | 19.2987 |
| 3.0000 | 0.4996 | 0.5003 | 0.7066 | 1.5656 | 1.5799 | 2.2171 | 24.2192 | 8.4915 | 42.5155 | 3.6870 | 19.2885 |
| 4.0000 | 0.5000 | 0.5001 | 0.7070 | 1.5608 | 1.5840 | 2.2250 | 19.6599 | 10.2282 | 45.0699 | 3.7106 | 19.3078 |
| 5.0000 | 0.6611 | 0.1574 | 0.8363 | 1.5788 | 1.5572 | 2.2046 | 15.4084 | 12.5044 | 49.5112 | 3.6906 | 19.2924 |
| 6.0000 | 0.5587 | 0.1583 | 0.9066 | 1.5416 | 1.5745 | 2.2237 | 11.2395 | 17.6782 | 46.7216 | 3.6995 | 19.3138 |
| 7.0000 | 0.4507 | 0.1921 | 0.9577 | 1.5714 | 1.5671 | 2.2229 | 8.6604 | 22.6915 | 44.5163 | 3.7051 | 19.3085 |
| 8.0000 | 0.3465 | 0.2592 | 0.9872 | 1.5787 | 1.5546 | 2.2119 | -4.1561 | 36.8078 | 68.5160 | 3.6986 | 19.2824 |
| 9.0000 | 0.2583 | 0.3458 | 0.9860 | 1.5676 | 1.5577 | 2.2126 | -5.0520 | 48.2597 | 61.3270 | 3.6861 | 19.2802 |
| 10.0000 | 0.1934 | 04510 | 0.9593 | 1.5610 | 1.5777 | 2.2239 | -1.5210 | 56.9179 | 51.3894 | 3.7080 | 19.3368 |
| 11.0000 | 0.1592 | 0.5609 | 0.9075 | 1.5677 | 1.5934 | 2.2148 | 5.3406 | 61.8023 | 41.0384 | 3.7071 | 19.2966 |
| 12.0000 | 0.1572 | 0.6613 | 0.8355 | 1.5741 | 1.5633 | 2.2236 | 13.7542 | 65.6280 | 32.5067 | 3.7068 | 19.3039 |
| 13.0000 | 0.1897 | 0.7485 | 0.7514 | 1.5838 | 1.5616 | 2.2162 | 24.0519 | 67.8979 | 25.2036 | 3.6916 | 193039 |
| 14.0000 | 0.2524 | 0.8110 | 0.6629 | 1.5693 | 1.5749 | 2.2135 | 35.5814 | 65.4718 | 19.3605 | 3.7016 | 19.2947 |
| 15.0000 | 0.3383 | 0.8426 | 0.5787 | 1.5746 | 1.5987 | 2.2084 | 45.5918 | 57.1092 | 16.3375 | 3.6940 | 19.2983 |
| 16.0000 | 0.4994 | 0.4994 | 0.7073 | 1.5866 | 1.5705 | 2.2392 | 55.0649 | 48.6262 | 15.8842 | 3.6966 | 19.3157 |
| 17.0000 | 0.4980 | 0.4988 | 0.7074 | 1.5697 | 1.5577 | 2.2220 | 62.8859 | 38.6791 | 18.1526 | 3.6981 | 19.3062 |
| 18.0000 | 0.4999 | 0.5000 | 0.7071 | 1.5618 | 1.5759 | 2.2290 | 66.2100 | 27.1726 | 23.2553 | 3.7191 | 19.3070 |
| 19.0000 | 0.4995 | 0.5000 | 0.7071 | 1.5645 | 1.5440 | 2.2160 | 65.9627 | 16.4580 | 30.2717 | 3.7027 | 19.3093 |
| 20.0000 | 0.5008 | 0.4999 | 0.7073 | 1.5809 | 1.5786 | 2.2285 | 64.4436 | 7.2734 | 38.9060 | 3.7100 | 19.3121 |
| 21.0000 | 0.4991 | 0.5003 | 0.7067 | 1.5595 | 1.5510 | 2.2249 | 29.9377 | 9.8916 | 34.6023 | 3.7096 | 19.2960 |
| 22.0000 | 0.5001 | 0.4999 | 0.7072 | 1.5773 | 1.5658 | 2.2270 | 31.5128 | 6.5065 | 41.7788 | 3.7076 | 19.2933 |
| 23.0000 | 0.4980 | 0.5016 | 0.7043 | 1.5544 | 1.5654 | 2.2138 | 23.7765 | 8.8506 | 41.8739 | 3.7016 | 19.2967 |
| 24.0000 | 0.4999 | 0.5002 | 0.7067 | 1.5787 | 1.5701 | 2.2318 | 19.9744 | 9.8257 | 45.9946 | 3.6852 | 19.2953 |
| 25.0000 | 0.5000 | 0.5000 | 0.7070 | 1.5708 | 1.5840 | 2.2203 | 14.8964 | 14.3105 | 43.2917 | 3.6979 | 19.2977 |
| 26.0000 | 0.5001 | 0.5001 | 0.7061 | 1.5677 | 1.5855 | 2.2159 | 11.5285 | 17.9672 | 44.3395 | 3.7077 | 19.3067 |
| 27.0000 | 0.4998 | 0.5000 | 0.7077 | 1.5607 | 1.5570 | 2.2109 | 8.6505 | 22.6944 | 44.5505 | 3.6967 | 19.2938 |
| 28.0000 | 0.5009 | 0.4993 | 0.7050 | 1.5711 | 1.5575 | 2.2267 | 8.6355 | 26.8102 | 39.1249 | 3.6963 | 19.2887 |
| 29.0000 | 0.5008 | 0.4990 | 0.7057 | 1.5566 | 1.5674 | 2.2197 | 8.5303 | 31.5145 | 37.0562 | 3.6806 | 19.3029 |
| 30.0000 | 0.5007 | 0.4987 | 0.7060 | 1.5727 | 1.5555 | 2.2112 | 10.3081 | 35.1426 | 33.4376 | 3.7108 | 19.3062 |

| | | | | | | | | | | | |
|---|---|---|---|---|---|---|---|---|---|---|---|
| Amx, Amy, Amz - Acceleration in Local system (g=9.8 m/sec²) Omgx, Omgy, Omgz - Angular velocity in Local system (rad/sec) Bmx, Bmy, Bmz - Magnetic field in Local system (micro Tesla) Vv - Magnetic Bridge voltage Temp - Temperature of sensor (celcius) | | | | | | | | | | | |

**Table 2**

| Time | Ksi | Fi | Theta | Axg | Ayg | Azg | Vxg | Vyg | Vzg | Pxg | Pyg | Pzg | Bxg | Byg | Bzg | Btc |
|---|---|---|---|---|---|---|---|---|---|---|---|---|---|---|---|---|
| 1.0000 | 0.7854 | 0.7847 | 0.7854 | -0.0013 | -0.0013 | 0.9987 | 0 | 0 | 0 | 0 | 0 | 0 | 16.1425 | 9.7038 | 47.2823 | 50.8956 |
| 2.0000 | 1.0996 | 0.7843 | 0.7846 | -0.0009 | -0.0009 | 0.9991 | -0.0012 | -0.0012 | -0.0012 | -0.0001 | -0.0001 | -0.0001 | 14.2604 | 7.8216 | 45.4001 | 48.2256 |
| 3.0000 | 1.4137 | 0.7863 | 0.7850 | -0.0003 | -0.0003 | 0.9997 | -0.0021 | -0.0021 | -0.0021 | -0.0002 | -0.0002 | -0.0002 | 16.2692 | 9.8304 | 47.4089 | 51.0777 |
| 4.0000 | 1.7279 | 0.7857 | 0.7846 | 0.0003 | 0.0003 | 1.0003 | -0.0024 | -0.0024 | -0.0024 | -0.0005 | -0.0005 | -0.0005 | 16.1097 | 9.6710 | 47.2495 | 50.8484 |
| 5.0000 | 2.0420 | 0.7852 | 0.7866 | 0.0003 | 0.4000 | 1.0003 | -0.0021 | -0.0021 | -0.0021 | -0.0007 | -0.0007 | -0.0007 | 16.4822 | 10.0435 | 47.6220 | 51.3847 |
| 6.0000 | 2.3562 | 0.7865 | 0.7858 | -0.0001 | 0.4000 | 0.9999 | -0.0019 | 0.3903 | -0.0019 | -0.0009 | 0.0187 | -0.0009 | 14.5166 | 8.0778 | 45.6563 | 48.5848 |
| 7.0000 | 2.6704 | 0.7829 | 0.7870 | 0.0007 | 0.4000 | 1.0007 | -0.0020 | 0.7827 | -0.0020 | -0.0011 | 0.0774 | -0.0011 | 13.5909 | 7.1521 | 44.7306 | 47.2937 |
| 8.0000 | 2.9845 | 0.7861 | 0.7836 | -0.0004 | 0.4000 | 0.9996 | -0.0014 | 1.1751 | -0.0014 | -0.0013 | 0.1753 | -0.0013 | 31.1304 | 24.6916 | 62.2701 | 73.8670 |
| 9.0000 | 3.2987 | 0.7849 | 0.7851 | -0.0011 | 0.4000 | 0.9989 | -0.0018 | 1.5675 | -0.0018 | -0.0014 | 0.3124 | -0.0014 | 33.1914 | 26.7526 | 64.3311 | 77.1743 |
| 10.0000 | 3.6128 | 0.7854 | 0.7855 | 0.0018 | 0.4000 | 1.0018 | -0.0028 | 1.9599 | -0.0028 | -0.0016 | 0.4887 | -0.0016 | 32.0739 | 25.6351 | 63.2136 | 75.3781 |
| 11.0000 | 3.9270 | 0.7855 | 0.7866 | -0.0013 | 0.4000 | 0.9987 | -0.0011 | 2.3523 | -0.0011 | -0.0018 | 0.7044 | -0.0018 | 33.1551 | 26.7164 | 64.2949 | 77.1159 |
| 12.0000 | 4.2412 | 0.7855 | 0.7862 | 0.0020 | 0.4000 | 1.0020 | -0.0023 | 2.7447 | -0.0023 | -0.0020 | 0.9592 | -0.0020 | 31.3579 | 24.9192 | 62.4977 | 74.2310 |
| 13.0000 | 4.5553 | 0.7853 | 0.7839 | -0.0003 | 0.4000 | 0.9997 | -0.0003 | 3.1371 | -0.0003 | -0.0021 | 1.2533 | -0.0021 | 31.7916 | 25.3528 | 62.9313 | 74.9254 |
| 14.0000 | 4.8695 | 0.7846 | 0.7865 | 0.0005 | 0.4000 | 1.0005 | -0.0007 | 3.5295 | -0.0007 | -0.0022 | 1.5866 | -0.0022 | 32.4964 | 26.0577 | 63.6362 | 76.0564 |
| 15.0000 | 5.1836 | 0.7864 | 0.7859 | -0.0005 | 0.4000 | 0.9995 | -0.0001 | 3.9219 | -0.0001 | -0.0022 | 1.9592 | -0.0022 | 33.0874 | 26.6487 | 64.2272 | 77.0068 |
| 16.0000 | 5.4978 | 0.7847 | 0.7857 | -0.0014 | -0.0014 | 0.9986 | -0.0007 | 4.3143 | -0.0007 | -0.0023 | 2.3710 | -0.0023 | 34.3117 | 27.8729 | 65.4514 | 78.9815 |
| 17.0000 | 5.8119 | 0.7865 | 0.7842 | 0.0001 | 0.0001 | 1.0001 | -0.0020 | 4.3129 | -0.0020 | -0.0024 | 2.8023 | -0.0024 | 31.5224 | 25.0836 | 62.6621 | 74.4943 |
| 18.0000 | 6.1261 | 0.7855 | 0.7844 | 0.0002 | 0.0002 | 1.0002 | -0.0019 | 4.3130 | -0.0019 | -0.0026 | 3.2336 | -0.0026 | 32.0317 | 25.5930 | 63.1715 | 75.3104 |
| 19.0000 | 6.4403 | 0.7870 | 0.7854 | -0.0002 | -0.0002 | 0.9998 | -0.0017 | 4.3132 | -0.0017 | -0.0028 | 3.6650 | -0.0028 | 33.2424 | 26.8037 | 64.3822 | 77.2565 |
| 20.0000 | 6.7544 | 0.7846 | 0.7858 | 0.0007 | 0.0007 | 1.0007 | -0.0019 | 4.3130 | -0.0019 | -0.0030 | 4.0963 | -0.0030 | 31.5655 | 25.1267 | 62.7052 | 74.5632 |
| 21.0000 | 7.0686 | 0.7832 | 0.7868 | 0.0001 | 0.0001 | 1.0001 | -0.0013 | 4.3137 | -0.0013 | -0.0031 | 4.5276 | -0.0031 | 16.6385 | 10.1997 | 47.7782 | 51.6104 |
| 22.0000 | 7.3827 | 0.7840 | 0.7861 | -0.0011 | -0.0011 | 0.9989 | -0.0011 | 4.3138 | -0.0011 | -0.0032 | 4.9590 | -0.0032 | 14.8018 | 8.3630 | 45.9415 | 48.9863 |
| 23.0000 | 7.6969 | 0.7846 | 0.7853 | 0.0003 | 0.0003 | 1.0003 | -0.0022 | 4.3127 | -0.0022 | -0.0034 | 5.3903 | -0.0034 | 13.8695 | 7.4308 | 45.0093 | 47.6803 |
| 24.0000 | 8.0111 | 0.7860 | 0.7857 | -0.0001 | -0.0001 | 0.9999 | -0.0019 | 4.3131 | -0.0019 | -0.0036 | 5.8216 | -0.0036 | 16.9017 | 10.4629 | 48.0414 | 51.9915 |
| 25.0000 | 8.3252 | 0.7849 | 0.7851 | 0.0005 | 0.0005 | 1.0005 | -0.0020 | 4.3129 | -0.0020 | -0.0038 | 6.2529 | -0.0038 | 15.8236 | 9.3849 | 46.9634 | 50.4383 |
| 26.0000 | 8.6394 | 0.7835 | 0.7848 | -0.0003 | -0.0003 | 0.9997 | -0.0015 | 4.3134 | -0.0015 | -0.0040 | 6.6842 | -0.0040 | 16.2448 | 9.8060 | 47.3845 | 51.0426 |
| 27.0000 | 8.9535 | 0.7851 | 0.7851 | 0.0004 | 0.0004 | 1.0004 | -0.0018 | 4.3131 | -0.0018 | -0.0042 | 7.1155 | -0.0042 | 14.8041 | 8.3653 | 45.9438 | 48.9896 |
| 28.0000 | 9.2677 | 0.7836 | 0.7848 | -0.0004 | -0.0004 | 0.9996 | -0.0014 | 4.3135 | -0.0014 | -0.0043 | 7.5469 | -0.0043 | 15.5882 | 9.1494 | 46.7279 | 50.1019 |
| 29.0000 | 9.5819 | 0.7849 | 0.7864 | -0.0001 | -0.0001 | 0.9999 | -0.0019 | 4.3131 | -0.0019 | -0.0045 | 7.9782 | -0.0045 | 13.8282 | 7.3895 | 44.9680 | 47.6229 |
| 30.0000 | 9.8960 | 0.7837 | 0.7854 | -0.0013 | -0.0013 | 0.9987 | -0.0020 | 4.3129 | -0.0020 | -0.0047 | 8.4095 | -0.0047 | 14.8412 | 8.4024 | 45.9809 | 49.0419 |

From time steps 8 to 20 the magnetic field cannot be used to calculate the orientation since the measured field is more than 10% (threshold set in this Example) greater than the local field of 49.6 microTesla (earth magnetic field).

From time step 0-4 and 16-30 Atot=Azg =1g =9.8 m/sec². Thus, here the direction of the gravity field is known and the orientation (euler angles)= (ksi, fi, theta) of the unit can be determined, and reset the drift. At the other time steps it is necessary to rely on the calculated values.
Axg,Ayg,Azg: composants of the total acceleration (including the acceleration of gravity in the global reference system).
Vxg,Vyg,Vzg: composants of calculated velocity in the global reference system. Pxg,Pyg,Pzg: composants of position in the global reference system.
Bxg, Byg, Bzg: composants of magnetic field in the global reference system (micro Tesla).

### EXAMPLE 2

### Plotting of ground contact / accumulating every ground contact

By placing a unit on each foot the ground contact can be extracted by accumulating, when each foot is not accelerating downwards anymore. At that point the acceleration is equal zero and the downwards movement is changed to an upward movement. This method requires the individual to touch ground with the heel and fore foot (the whole foot touches ground), which is normal for most individuals, but may not apply to all individuals.

Extracting the ground contact can be used to correctly benchmark performance according to the ground covered, hereby equal data can be compared. E.g. running on a beach or across a field is much harder than running on the pavement. The efficient energy use can also be calculated for cross country runners or soldiers taking different roads, but using more energy and covering harder conditions than others travelling the same distance.

GPS can correctly measure the direction on a map, by extracting the ground contact using the method above, the GPS coordinates in connection with the ground contact can provide even more valuable information about the route travelled.

### Output parameters

Each time set contains the following parameters:
Am = Accelerometer
Omg = Gyroscope
Bm = Magnetometer
Vv = Reference voltage
Temp = Temperature
Time = Time interval

Output string:
Time, Amx, Amy, Amz, Omgx, Omgy, Omgz, Bmx, Bmy, Bmz, Vv, and Temp

### Using the data material of the unit in 3D programs

3D programs are used to illustrate and analysis movement of traditional analysis methods such as capture of movement data via several motion cameras focusing on markers placed on an individual.

Data captured by the unit described in this patent can be transformed to the public domain C3D, which most 3D programs are able to read, everything need to transform data into the C3D format can be found on www.c3d.org and is public domain. However the unit is not able to extract the initial position in 3D, therefore one of the following methods can be used to extract the position of the unit on the body of a person, this is even more relevant when using more than one unit, since the movement can be correctly animated in 3D space by knowing the initial position:
1) A handheld or a standing 3D scanner e.g. laser scanner can be used.
2) A special pattern of movement can be used. The person has only placed units, so that these can be pressed against the floor at the same time by lying down. The person stands up and hereby respective units can a placed in regard to the X and Y axis by motioning them with the respective units. If the person stands on a tum table and rotates 360 degrees, the position of the units in regard to the Z axis can be extracted. The position in 3D space can hereby be extracted.
3) Markers can be mounted upon the unit to extract the position via motion capture cameras using existing technology.
4) A mathematical model can be setup by using geometry and physical measurements in regard to distance and angles between units and ground. The physical measurements can be measured and typed into the model. The model can generate the initial coordinates of the respective units in 3D space.
5) A computer model in 2D or 3D of the object to be analysed e.g. a human, could be shown on a computer/PDA or likewise. On the computer model the user should be able to point out, where the unit(s) are placed as initial position(s). By using such a computer model the initial position of a unit or more units can be calculated.

### EXAMPLE 3

### Mounting the unit on the skin of a person.

In high impact sports involving high levels of sweat production it is a major task to ensure that the unit remains in appropriate contact with the skin, since the adhesive used to mount the unit on the skin is negatively influenced by the salty and aqueous environment generated on the skin surface due to the increased sweat production. This results in the adhesive losing its grip on the skin of the person.

It is therefore contemplated by the present inventors to provide a viable solution to this problem. It has been found that maintained skin contact of the unit is highly dependent on the form of the adhesive patches applied to the skin side of unit of the present invention.

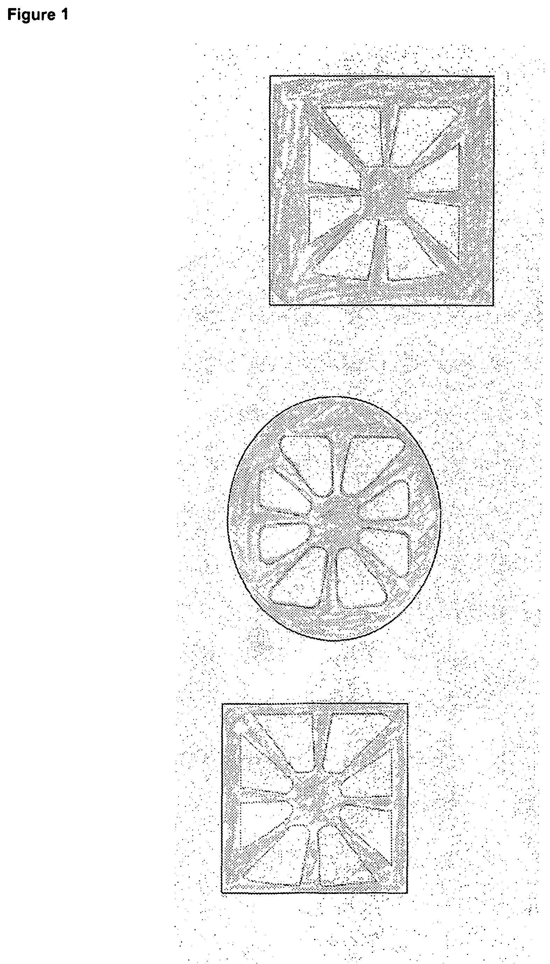

After a period with large amounts of sweat the adhesive stops to work in the red area. Adhesive disc (can be mounted underneath the unit to increase the flow of sweat away from the bottom of the unit. Examples of applicable patch designs are provided in Figure 1.

### EXAMPLE 4

### Description of print

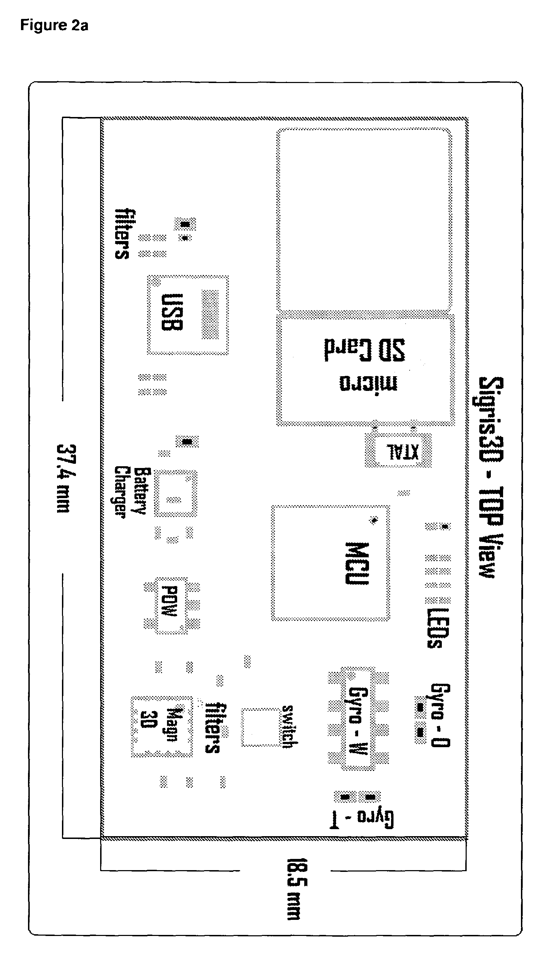

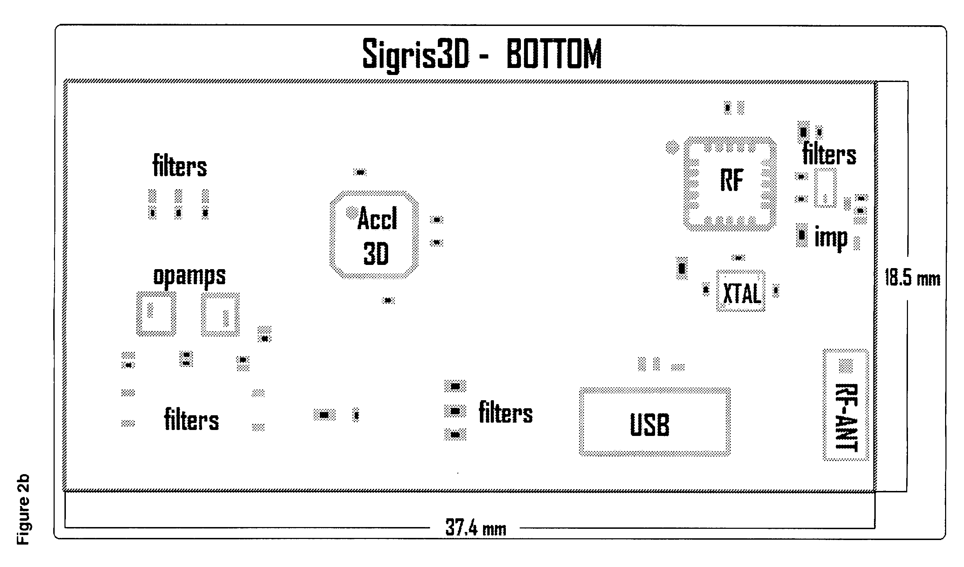

Reference is made to Figure 2a and Figure 2b.
Gyro Epson XV3500CB , (3 pc single axe (W,O,T)) Sensitivity 0.67mV/deg/s,
Magn 3D (3-axe mag-fieldsensor) HMC1043 Honeywelll Sensitivity 1mV/V/Gauss
ACCL 3D accelerometer ADXL330KCPZ Sensitivity 300mV/g
MCU MSP430 16 MHz Texas Instrument
SD Card Sandisk 2 GB
USB TUSB3410TQFN Texas Instrument: USB connection between USBport on computer and data retrieved of microprocessor MCU on SD Card
RF CC2500 (Texas Instrument: Wireless transmitter and receiver.
Batt Charger Maxim MAX8808 Battery charger
Opamp Operation amplifier
Filter Electronically filters
RF-ant RF-antenna
X-tal Crystal for frequency ref.
Pow Power management
LED light emitting diodes for visual communication of operational states
Component without names (capasitors and resistors)

The ADC converter of the microprocessor samples the signals of the individual sensors and converts analogue signals to digital signals. The sampling may be performed at adjustable frequencies, e.g. 1 kHz. The signals are stored on an SD-card. Subsequently the measurements are transmitted through the USB unit to a USB port on a computer for further data processing and analysis; alternatively the data are wirelessly transmitted by the RF unit for displaying the actual movement upon immediate processing in a computer.

## Claims

1. An integrated unit for determining the position of a body part in space, comprising:
• an accelerometer component operative to perform acceleration measurements along 3 orthogonal axes;
• a gyroscopic component operative to measure rotational velocity along said 3 orthogonal axes;
• a magnetometer component operative to perform magnetic measurements along said 3 orthogonal axes;
• a data memory unit connected to said components, said data memory unit used for receiving and storing data from said components;
• a power supply; and
• timer for synchronizing the operation of the components;
**characteized in that** said components and data memory unit are embedded in a polymer matrix, which is provided with means for attaching the unit to the skin of a human.

2. An integrated sensor unit according to claim 1, wherein the means for attaching the unit to the skin of a human is an adhesive layer.

3. An integrated sensor unit according to claim 1 or 2, wherein the accelerometer component, the gyroscopic component, and magnetometer component, each comprises at least three sensors of the corresponding type.

4. An integrated sensor unit according to any one of claims 1 to 3, wherein the sensor unit communicates with a foot sensor for placement under each of the patient's feet, wherein said foot sensors are connected to said data memory unit and are operative to sense pressure.

5. An integrated sensor unit according to any one of claims 2 to 4, wherein the polymer matrix and the adhesive for attaching the unit to the skin are silicone based.

6. An integrated sensor unit according to any one of claims 1 to 5, wherein the data memory unit stores said data from said sensors for transference to a data processing unit, said data being transmitted to said data processing unit from said data memory unit wirelessly, through a cable or with a memory card.

## Patentansprüche

1. Integrierte Einheit zur Positionsbestimmung eines Körperteils im Raum, umfassend:
• ein Bauteil zur Beschleunigungsmessung, das im Betrieb Beschleunigungsmessungen entlang 3 orthogonaler Achsen durchführt;
• ein gyroskopisches Bauteil, das im Betrieb die Rotationsgeschwindigkeit entlang der 3 orthogonalen Achsen misst;
• ein Bauteil zur Messung der Magnetfeldstärke, das im Betrieb magnetische Messungen entlang der 3 orthogonalen Achsen durchführt;
• eine mit diesen Bauteilen verbundene Datenspeichereinheit, wobei die Datenspeichereinheit zum Empfangen und Speichern der Daten von diesen Bauteilen verwendet wird;
• eine Stromversorgung; und
• ein Zeitgeber zum Synchronisieren des Betriebs der Bauteile;
**dadurch gekennzeichnet, dass** die Bauteile und die Datenspeichereinheit in eine Polymermatrix eingebettet sind, die mit einem Mittel zum Anbringen der Einheit auf die Haut eines Menschen versehen ist.

2. Integrierte Sensoreinheit nach Anspruch 1, wobei das Mittel zum Anbringen der Einheit auf die Haut eines Menschen eine Klebschicht ist.

3. Integrierte Sensoreinheit nach Anspruch 1 oder 2, wobei das Bauteil zur Beschleunigungsmessung, das gyroskopische Bauteil und das Bauteil zur Messung der Magnetfeldstärke jeweils wenigstens drei Sensoren des entsprechenden Typs umfassen.

4. Integrierte Sensoreinheit nach einem der Ansprüche 1 bis 3, wobei die Sensoreinheit mit einem Fußsensor zur Anordnung unter jedem der Füße des Patienten in Verbindung steht, wobei die Fußsensoren mit der Datenspeichereinheit verbunden sind und im Betrieb den Druck fühlen.

5. Integrierte Sensoreinheit nach einem der Ansprüche 2 bis 4, wobei die Polymermatrix und der Klebstoff zum Anbringen der Einheit auf die Haut auf Silikon basieren.

6. Integrierte Sensoreinheit nach einem der Ansprüche 1 bis 5, wobei die Datenspeichereinheit die Daten von den Sensoren zur Übertragung an eine Datenverarbeitungseinheit speichert, wobei die Daten von der Datenspeichereinheit drahtlos, über ein Kabel oder mit einer Speicherkarte an die Datenverarbeitungseinheit übermittelt werden.

## Revendications

1. Module intégré pour déterminer la position d'une partie corporelle dans l'espace, comprenant :
• un composant d'accéléromètre fonctionnant pour réaliser des mesures d'accélération le long de 3 axes orthogonaux ;
• un composant gyroscopique fonctionnant pour mesurer une vitesse de rotation le long desdits 3 axes orthogonaux ;
• un composant de magnétomètre fonctionnant pour réaliser des mesures magnétiques le long desdits 3 axes orthogonaux ;
• un module de mémoire de données connecté auxdits composants, ledit module de mémoire de données étant utilisé pour recevoir et stocker des données provenant desdits composants ;
• une alimentation électrique ; et
• une minuterie pour synchroniser le fonctionnement des composants ; **caractérisé en ce que**
lesdits composants et le module de mémoire de données sont encastrés dans une matrice polymère qui est pourvue d'un moyen pour relier le module à la peau d'un homme.

2. Module de capteur intégré selon la revendication 1, dans lequel le moyen pour relier le module à la peau d'un homme est une couche adhésive.

3. Module de capteur intégré selon la revendication 1 ou 2, dans lequel le composant d'accéléromètre, le composant gyroscopique et le composant de magnétomètre comprennent chacun au moins trois capteurs du type correspondant.

4. Module de capteur intégré selon l'une quelconque des revendications 1 à 3, dans lequel le module de capteur communique avec un capteur de pied pour le placement en dessous de chacun des pieds du patient, dans lequel lesdits capteurs de pied sont connectés audit module de mémoire de données et sont opérationnels pour capter la pression.

5. Module de capteur intégré selon l'une quelconque des revendications 2 à 4, dans lequel la matrice polymère et l'adhésif pour relier le module à la peau sont à base de silicone.

6. Module de capteur intégré selon l'une quelconque des revendications 1 à 5, dans lequel le module de mémoire de données stocke lesdites données provenant desdits capteurs pour le transfert à un module de traitement de données, lesdites données étant transmises audit module de traitement de données depuis ledit module de mémoire de données sans fil, par un câble ou avec une carte mémoire.
